# FASCICULE DE BREVET EUROPEEN

(11) **EP 1 349 639 B1**
(45) Date de publication et mention de la délivrance du brevet: **03.12.2008**
(21) Numéro de dépôt: 01911803.3
(22) Date de dépôt: 05.01.2001
(51) Int. Cl.: B01D 59/30, C07C 237/20, B01J 45/00, B01D 59/22

(54) **PROCEDE DE SEPARATION D'ISOTOPES**
VERFAHREN ZUR TRENNUNG VON ISOTOPEN
METHOD OF SEPARATING ISOTOPES

(43) Date de publication de la demande: 08.10.2003
(73) Titulaire: Areva NP, 92400 Courbevoie (FR)
(72) Inventeur: LEMAIRE, Marc, 69100 Villeurbanne (FR); FOOS, Jacques, F-91400 Orsay (FR); GUY, Alain, F-77135 Pontcarre (FR); CHITRY, Frédéric, F-92140 Clamart (FR); PELLET-ROSTAING, Stéphane, F-69100 Villeurbanne (FR); VIGNEAU, Olivier, F-91240 St. Michel-sur-Orge (FR)
(74) Mandataire: Colombet, Alain André
(86) Numéro de dépôt international: PCT/FR2001/000037
(87) Numéro de publication internationale: WO 2002/053269

(56) Documents cités:
- EP-A- 0 142 126
- FR-A- 881 316
- FR-A- 1 583 034
- FR-A- 2 214 509
- US-A- 2 835 687
- US-A- 3 953 568
- US-A- 4 600 566
- WHITWORTH T M ET AL: "ISOTOPIC FRACTIONATION AND OVERALL PERMEATION OF LITHIUM BY A THIN-FILM COMPOSITE POLYAMIDE REVERSE OSMOSIS MEMBRANE" JOURNAL OF MEMBRANE SCIENCE, ELSEVIER SCIENTIFIC PUBL.COMPANY. AMSTERDAM, NL, vol. 88, no. 2/3, 16 mars 1994 (1994-03-16), pages 231-241, XP000488346 ISSN: 0376-7388

## Description

L'invention est rotative à la séparation d'isotopes d'alcalino-terreux, d'éléments de transition et de métaux lourds ayant une masse atomique inférieure à 209. Elle concerne plus particulièrement la séparation des isotopes des terres rares ou lanthanides. L'invention a donc trait à un procédé permettant de séparer entre eux les isotopes de ces éléments.

Certains isotopes des lanthanides sont utilisés comme poison neutronique ou absorbeur de neutrons dans les réacteurs nucléaires. C'est notamment le cas du gadolinium (Gd) et de l'erbium (Er). Tous les isotopes du gadolinium (152, 154, 155, 156, 157, 158, 160) et de l'erbium (162, 164, 166, 167, 168, 170) n'ont pas le même intérêt et les industriels cherchent à isoler les isotopes les plus favorables. Les isotopes 155 et 157 du gadolinium et l'isotope 167 de l'erbium sont les isotopes ayant les meilleurs pouvoirs d'absorption de neutrons et sont les isotopes de choix comme neutrophages dans les éléments de combustible pour centrale nucléaire.

Pour plus d'informations sur les isotopes des lanthanides, voir Handbook of Chemistry and Physics, 73ème édition, 1992-1993.

La séparation des isotopes d'un même élément est l'un des problèmes techniques les plus difficiles à résoudre quelle que soit l'échelle de séparation choisie. Elle l'est d'autant plus que la différence de masse relative entre les isotopes est faible, e.g. entre 1 et 2% pour les lanthanides.

La séparation des isotopes d'éléments comme Ca ou Na en phase liquide (phase aqueuse ou organique) à l'aide d'agents complexants a été effectuée pour la première fois par les équipes de Jepson et DeWitt (J. Inorg. Nucl. Chem. 38, 1175, 1976) ainsi que de Heumann et Schiefer (Z. Naturforsch., 36b, 566, 1981). Les techniques employées faisaient appel à l'extraction liquide-liquide et à des résines échangeuses d'ions à l'aide de complexants spécifiques tels que les éthers-couronnes (dicylclohexano[18]couronne-6) ou les criptands (criptand[2.2.2]).

La séparation des isotopes de terres rares par résine échangeuse d'ions (chromatographie) en phase liquide impliquant des résines échangeuses d'ions et une solution éluante contenant un ligand des isotopes, a aussi été proposée. Ainsi, EP-A-173 484 propose une telle technique pour la séparation des isotopes du gadolinium en utilisant de 5 à 30, de préférence de 20 à 30 colonnes contenant soit une résine échangeuse d'anions, soit une résine échangeuse de cations comme phase stationnaire. Dans le premier cas, l'éluant contient du nitrate d'ammonium dans du méthanol aqueux et dans le deuxième cas, de l'EDTA. Dans le même ordre d'idées, on peut citer J. Chen et al., Journal of Nuclear Science and Technology, 1992, 29 (11) :1086-1092, et I.M. Ismail et al., J. Chromato. A, 1998, 808 : 185-191.

Pour des raisons essentiellement liées aux difficultés de contrôle de l'élution des pics d'isotopes, WO-A-96 00124 a cherché à perfectionner la séparation des isotopes du Gd sur résine échangeuse d'ions. La méthode décrite fait alors appel à une phase mobile formée de préférence d'un acide aqueux dont le préféré est l'acide nitrique. Une méthode similaire est proposée par WO-A-96 00123 pour la séparation des isotopes de l'erbium.

D'autres auteurs ont proposé un système red-ox par échange chimique dans des systèmes d'extraction liquide-liquide avec ligands de type HDEHP ou TBP, pour la séparation d'isotopes de l'europium et du cérium (W. Dembinski et T. Mioduski, Journal of Radioanalytical and Nuclear Chemistry, Letters, 1995, 199(2) : 159-171 ; W. Dembinski et al., Journal of Radioanalytical and Nuclear Chemistry, Articles, 1991, 149(1) : 169-176).

FR-A-2 214 509 a proposé de séparer les isotopes 44 et 40 du calcium par un procédé d'extraction liquide-liquide basé sur l'utilisation d'éthers-couronnes et de solvant de type eau-alcool ou de solvant chloré.

La séparation des isotopes est donc un enjeu considérable. Toutefois, les différentes techniques présentent des inconvénients, e.g. la complexité des moyens à mettre en oeuvre ; le coût et les dimensions des installations ; la dépense énergétique ; la production de sous-produits liquides ou solides plus ou moins toxiques ; l'utilisation de solvants pose le problème de leur séparation et de leur retraitement, en vue de leur recyclage et de la protection de l'environnement.

L'invention a donc pour objectif de fournir un procédé permettant de séparer efficacement entre eux des isotopes d'un même élément, appartenant à la catégorie des alcalino-terreux, des éléments de transition et des métaux lourds ayant une masse atomique inférieure à 209, en particulier des lanthanides.

Un autre objectif de l'invention est de fournir un tel procédé qui permette l'enrichissement d'un élément en un ou plusieurs isotopes d'intérêt.

Un autre objectif encore de l'invention est de fournir un tel procédé permettant aisément d'ajuster le degré de séparation ou d'enrichissement.

Un autre objectif encore de l'invention est de fournir un tel procédé qui soit simple à mettre en oeuvre, qui ne nécessite pas d'installation trop volumineuse et soit économique à l'usage comme à l'installation.

Un autre objectif encore de l'invention est de fournir un tel procédé qui puisse permettre de limiter les volumes de déchets liquides et solides et de ne pas générer de déchets toxiques.

Un autre objectif encore de l'invention est de fournir un tel procédé qui soit facile à mettre en oeuvre et qui permette de limiter les dépenses énergétiques.

Un autre objectif encore de i'invention est de fournir un tel procédé qui puisse fonctionner aussi bien en discontinu qu'en continu.

Un autre objectif encore de l'invention est de fournir un tel procédé qui autorise des débits d'élution élevés, notamment supérieurs à ceux obtenus dans l'art antérieur.

Ces objectifs, ainsi que d'autres, sont atteints par le procédé de la revendication 1. L'utilisation d'un moyen de séparation approprié permet alors d'une part de retenir un rétentat comprenant les isotopes complexés par le ligand, ce qui se traduit par un enrichissement du rétentat, par rapport à la solution initiale, en isotope(s) se complexant de manière préférentielle, et d'autre part de laisser passer ou éluer un perméat comprenant les isotopes qui ne se sont pas complexés, ce qui se traduit par un perméat ou éluat enrichi en isotopes ne se complexant pas de façon préférentielle, à savoir isotopes ne se complexant pas et/ou isotopes se complexant plus faiblement.

Le métal à traiter se trouve en solution aqueuse sous forme de sel d'isotope, en particulier nitrate, sulfate, carbonate ou chlorure.

Le choix du ligand se porte sur un ligand organique, ce qui permet de concevoir et synthétiser des ligands selon un cahier des charges précis. En particulier, la taille du ligand peut être choisie de façon à optimiser l'opération de séparation entre complexe ligand-isotope et isotopes libres en fonction du moyen de séparation utilisé. Un autre paramètre est la charge ionique du ligand. Le ligand organique est choisi de façon à avoir le même nombre de charges négatives que l'isotope sous forme ionique a de charges positives.

L'invention est basée sur un constat surprenant : il est possible, avec un ligand approprié, de complexer préférentiellement un ou certains isotopes d'un élément, au détriment d'un ou plusieurs autres et ceci de manière reproductible, puis de séparer les isotopes complexés des isotopes non complexés.

Le ligand est sélectionné de manière à permettre d'enrichir soit le perméat, soit le rétentat en isotope intéressant, voire les deux lorsqu'il s'agit de séparer deux isotopes intéressants.

De préférence, le ligand est un polyacide aminé linéaire ou cyclique, en particulier linéaire, de préférence de formule (I)
suivante : 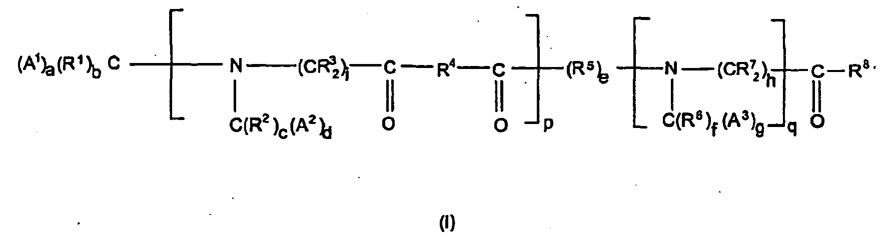 dans laquelle :
- a = 0 ou 1 et b = 2 ou 3
- c = 2 ou 3 et d = 0 ou 1
- e = 0 ou 1
- h = 1, 2 ou 3, de préférence 1 ou 2
- i = 1, 2 ou 3, de préférence 1 ou 2
- p = 0 à 3, de préférence 2
- q = 1 à 4, de préférence 2 ou 3
- f = 2 ou 3
- g = 0 ou 1
A¹, A², A³ sont identiques ou différents entre eux et correspondent à un groupement acide monovalent, de préférence sélectionné dans le groupe comportant :-COOR,
- PO₃R'₂, -SO₃R", avec R, R', R" = H ou cation, notamment un cation alcalin, de préférence le sodium ; on choisit de préférence un cation qui ne complexe pas le ligand ;
- les R₁ sont identiques ou différents entre eux et correspondent à :
   ∈ H,
   ∈ alkyle en C₁-C₁₀ ou,
   ∈ 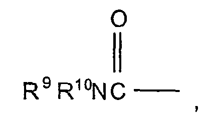 avec a = 0, b = 1 et R⁹, R¹⁰ identiques ou différents et correspondant chacun à l'hydrogène ou à un radical monovalent hydrophile sélectionné de préférence parmi les restes hydrocarbonés aminés et/ou (poly)hydroxylés et/ou alcoxylés et/ou (poly)étherifiés, ces restes étant de préférence du type (cyclo)alkyle, aralkyle, alkylaryle, (cyclo)alcényle, aralcényle, alcénylaryle, aryle ;
      R⁹, R¹⁰ correspondant plus préférentiellement encore chacun à un hydroxyalkyle en C₁-C₁₀, un alcoxyle en C₁-C₁₀, un polyol, avantageusement un saccharide hydrogéné ;
- les R² sont identiques ou différents entre eux,
   les R³ sont identiques ou différents entre eux,
   les R⁶ sont identiques ou différents entre eux,
   les R⁷ sont identiques ou différents entre eux,
   R², R³, R⁶, R⁷ sont identiques ou différents entre eux et correspondent à H ou à un alkyle en C₁-C₁₀;
- les R⁴ sont identiques ou différents entre eux et correspondent à un groupement divalent hydrophile sélectionné, de préférence, parmi les groupements aminés et/ou hydroxylés aromatiques, les groupements aminés et/ou hydroxylés aromatiques et alkyles, les groupements aminés et/ou hydroxylés aromatiques et (cyclo)alkylèniques, ou les groupements aminés et/ou hydroxylés (cyclo)alkyléniques ;
   ce groupement pouvant comporter des alcoxyles et/ou des (poly)éthers ;
- le groupement divalent R⁵ représentant un alkylène, de préférence CH₂, ou un groupement répondant à la même définition que R⁴ ; et/ou
- le groupement R⁸ correspondant à un hydroxyle, à A⁴ répondant à la même définition que A¹, A², A³, à l'hydrogène ou à -NR⁹R¹⁰ avec R⁹, R¹⁰ identiques ou différents entre eux et représentant un radical monovalent hydrophile sélectionné, de préférence, parmi les restes hydrocarbonés aminés et/ou (poly)hydroxylés et/ou alcoxylés et/ou (poly)étherifiés, ces restes étant de préférence du type (cyclo)-alkyle aralkyle, alkylaryle, (cyclo)alcényle, aralcényle, alcénylaryle, aryle ; R⁸ étant plus préférentiellement encore un hydroxyalkyle en C₁-C₁₀. un alcoxyle en C₁-C₁₀ ou un polyol, avantageusement un saccharide hydrogéné.

Tous les groupes ou radicaux monovalents ou divalents auxquels il est fait référence dans cette formule (1) peuvent être des alkyles ou des alcényles linéaires ou ramifiés pouvant comporter dans leur chaîne un ou plusieurs atomes d'oxygène à la place des atomes de carbone (e.g. alcoxy ou (poly)éther).

Dans cette même formule (I), on entend par groupe "aryle", un groupe dérivé d'un motif hydrocarboné aromatique comportant un ou plusieurs noyaux aromatiques pouvant être substitué ou non par des groupements OH, alkyle ou des hydroxyalkyles par enlèvement d'un atome d'hydrogène d'un des carbones du cycle ou par enlèvement d'un atome d'hydrogène d'un des carbones d'un substituant alkyle ou hydroxyalkyle. A titre d'exemples, on peut citer les groupes benzyles alcools ou hydroxyalkylphénols.

Dans cette formule (I), on entend également par groupe "cycloalkylène", un groupe divalent dérivé d'un hydrocarbocyclique substitué ou non par des chaînes alkyles ou hydroxyalkyles par enlèvement d'un atome d'hydrogène, un atome de carbone du cycle. A titre d'exemple, on peut citer le groupe cyclohexylène.

Par "hydrocarboné", on entend au sens de l'invention tout groupement comportant notamment des atomes de carbone et des atomes d'hydrogène.

Quand on fait référence à des alkyles, des alcoxyles ou des alcényles en C₁-C₁₀, il s'agit plus spécialement de radicaux en C₂, C₃, et/ou C₄.

Avantageusement les groupements hydrophiles qui peuvent représenter R⁹ et R¹⁰ sont des polyhydroxyalkyles, de préférence, des saccharides hydrogénés, plus préférentiellement encore, un reste sorbitol ou des chaînes polyéthers de préférence polyéthylèneglycol ou polypropylèneglycol.

Avantageusement, cette formule (I) englobe des polyacides aminés linéaires connus que sont l'EDTA et le DTPA (p = 0, q = 2 ou 3, b = 2, f = 2, A₁ = A₃ = COOH, R¹= R⁶ = R⁷= H, R⁵ = CH₂, R⁸ = OH, g = 1, e = 0, a = 1, h = 1 ou 2).

Suivant une variante, les ligands/complexants peuvent être des polyacides aminés cycliques comme, pas exemple, les DOTA qui sont des polyaminocarboxylates cycliques.

Dans une forme préférée de mise en oeuvre, le ligand/complexant est un produit de formule (I.1): 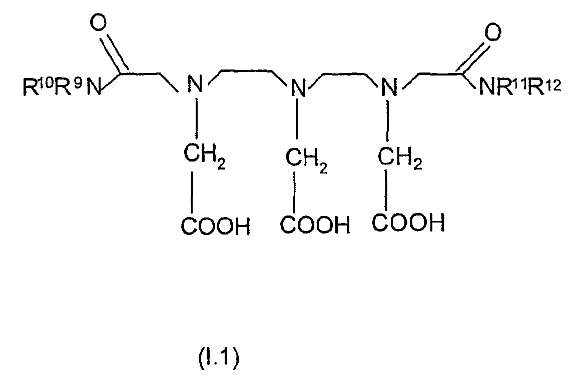 dans laquelle R⁹, R¹⁰, R¹¹, R¹² sont identiques ou différents entre eux et représentent chacun un radical monovalent hydrophile répondant à la même définition que celle donnée pour R⁹, R¹⁰, les radicaux éthanoyle, méthoxyéthyle, sorbitoyle étant plus spécialement préférés.

L'homme du métier peut déterminer les valeurs des variables et choisir les substituants pour concevoir des ligands adaptés aux isotopes à traiter.

Sur la base des informations divulguées, l'homme du métier est à même de définir des ligands appropriés pour la séparation de tel ou tel isotope en recourant à de simples expérimentations qui sont à la portée de ses compétences normales et qui peuvent consister par exemple à reproduire le procédé de l'invention avec un ligand donné, à procéder à la séparation et à analyser le perméat et le rétentat comme cela est notamment décrit dans les exemples.

Dans la première étape de ce procédé, on ajoute à la solution aqueuse à traiter le ligand hydrosoluble selon l'invention. La quantité de ligand ajoutée est de préférence telle qu'elle soit inférieure à un équivalent de ligand par atome d'isotope à séparer. Ces ligands forment des complexes de type 1:1.

Suivant une première modalité de l'invention, le moyen de séparation est une membrane de filtration, de préférence une membrane de nanofiltration. Le ligand et la membrane sont choisis de façon que la masse moléculaire du complexe ligand-isotope soit supérieure au seuil de coupure de la membrane, de façon à permettre une rétention aussi complète que possible des ions complexés.

Pour réaliser la séparation des isotopes, on fait circuler la solution aqueuse à traiter au voisinage de la membrane de nanofiltration et on applique une différence de pression entre les deux faces opposées de la membrane, de façon à recueillir un rétentat enrichi en isotope se complexant de manière préférentielle, et un perméat appauvri en cet isotope. La différence de pression entre les deux faces opposées de la membrane peut varier dans une large gamme, mais de bons résultats sont obtenus avec une différence de pression allant de 0,2 à 0,8 MPa.

Les membranes de nanofiltration susceptibles d'être utilisées dans le procédé de l'invention doivent présenter un seuil de coupure tel qu'elles laissent passer les ions non-complexés, et retiennent les ions complexés par les ligands de l'invention. Le seuil de coupure d'une membrane vis-à-vis d'un soluté neutre peut être défini comme la masse molaire minimale d'un composé nécessaire pour avoir un taux de rétention de ce composé à 90 %.

Selon l'invention, le seuil de coupure approprié pour la membrane sélectionnée peut être (en g/mol) de 100 à 5 000, de préférence de 200 à 2 000, et plus préférentiellement encore de 500 à 1 500. En pratique, le seuil de coupure peut être, par exemple, compris entre 200 et 2 000 g/mol.

Ces membranes peuvent être organiques, minérales ou organo-minérales. Elles comprennent avantageusement ou sont avantageusement constituées de polymères tels que les polyaramides, les polysulfones sulfonés, les polybenzimidazolones, les polyfluorures de vinylidène greffés ou non, les polyamides, les esters cellulosiques, les éthers cellulosiques ou les ionomères perfluorés, les associations de ces polymères et les copolymères obtenus à partir de monomères d'au moins deux de ces polymères. Pour plus de détails, l'homme du métier peut se référer à WO-A-92 06675 qui décrit des membranes de nanofiltration organominérales comprenant une couche active d'un polymère du type polysulfone, polybenzènimidazolone, polyfluorure de vinylidène greffé et ionomère perfluoré (nafion®) - seuil de coupure de 300 à 1 000 g. mol⁻¹ ; ou à FR-A-2 600 264 qui décrit des membranes organominérales comprenant un support poreux et organique et une membrane microporeuse en polymère organique tel que polysulfone, polyamide, ester cellulosique et éther cellulosique.

A titre d'exemples de membranes, on peut citer en particulier les membranes commercialisées par la firme OSMONICS sous les dénominations de SEPA MG-17, SEPA MW-15 et SEPA BQ-01, qui possèdent une perméabilité à l'eau bidistillée comprise entre 2 et 10 L.h⁻¹.m⁻². bar⁻¹ à 25° C.

On utilise de préférence la technique de filtration tangentielle, qui a l'avantage de limiter le phénomène d'accumulation des espèces retenues à la surface de la membrane et donc de permettre un fonctionnement en continu.

De préférence encore, on utilise des modules de filtration sous forme de tubes ou cylindres ou de plaques parallèles ou encore des membranes enroulées autour d'un tube ou cylindre perforé destiné à collecter le perméat. Ces modules peuvent être disposés en série et/ou en parallèle, avec éventuellement des membranes différentes dans certains modules.

A titre d'exemple, on peut citer la membrane commercialisée par Millipore sous la dénomination NANOMAX 50® qui est un module plan-spiralé dont la membrane est formée d'un support en polyester, d'une couche intermédiaire en polysulfone et d'une couche nanofiltrante en polyamide. Ses caractéristiques sont une forte rétention des ions multivalents et des composés non chargés de masse molaire M > 350 g/mol et une forte transmission des ions monovalents et des composés non chargés de masse molaire M < 100 g/mol.

Le pH du milieux aqueux, la différence de pression appliquée, la vitesse de circulation du rétentat et la température sont des paramètres ajustables.

Le pH est de préférence acide et compris entre 0 et 7 pour éviter la précipitation des hydroxydes de lanthanides aux pH élevés.

On peut travailler entre 0 et 50° C, et avantageusement à température ambiante (25° C) ou au voisinage de celle-ci, e.g. entre 20 et 35°C.

Les différences de pression et vitesse de circulation du rétentat sont avant tout fixées en fonction du débit recherché et des caractéristiques de la membrane, e.g. sa résistance à la pression. De simples essais permettent d'accéder aux conditions optimales.

On peut cependant préciser que la différence de pression peut avantageusement varier entre 0,2 et 1,5 MPa.

Après leur séparation des isotopes non complexés, les complexes ligand/isotope peuvent être traités à l'aide d'agent(s) de décomplexation approprié(s), de façon à recueillir, d'une part, les ligands et, d'autre part, le ou les isotopes.

Ainsi, les ions complexés peuvent, après filtration, être libérés ou décomplexés, par exemple en milieu basique et par précipitation de leur hydroxyde ou par passage sur résine échangeuse d'ions spécifique. Dans le cadre de cette étape, il est avantageux de prévoir conformément à l'invention une élimination du solvant - en l'occurrence de l'eau - par exemple par évaporation, pour permettre la récupération des ions séparés.

L'équipement requis pour la mise en oeuvre du procédé selon l'invention est relativement limité puisqu'il suffit d'un réacteur de complexation, d'une pompe et d'au moins une membrane de nanofiltration. A titre d'exemple, l'installation de base peut comprendre un réacteur de complexation, une pompe et un module de nanofiltration, e.g. tangentiel, conçu de façon que le rétentat, après son passage au voisinage de la membrane, soit recyclé en amont du module de filtration, de préférence dans le réacteur de complexation. Suivant une modalité particulière de l'invention, le réacteur peut être alimenté en continu ou semi-continu avec le ligand et le métal.

La nanofiltration peut avantageusement comprendre plusieurs étages, en série et/ou en parallèle, de manière à accroître le degré de séparation ou d'enrichissement, perméat et/ou rétentat étant soumis au nombre d'étapes de traitement et de nanofiltration requis par l'objectif à atteindre.

De même, des complexations-nanofiltratlons successives avec des ligands identiques ou différents peuvent être réalisées de manière à permettre la séparation de différents isotopes en plusieurs stades.

Comme les isotopes complexés, les isotopes décomplexés peuvent être à nouveau traités et nanofiltrés conformément à l'invention, et cela au moins une fois.

La nanofiltration n'a jamais été jusqu'à présente proposée pour séparer des isotopes selon l'invention, en particulier des isotopes de lanthanides.

L'utilisation de la nanofiltration présente de nombreux avantages par rapport aux techniques antérieures, notamment une faible consommation d'énergie puisqu'il est notamment possible de travailler à température ambiante, une bonne sélectivité, un bon débit d'élution, une grande souplesse d'utilisation, la possibilité de fonctionner de façon continue ou semi-continue et à grande échelle, un niveau d'investissement faible, une compacité élevée des étages séparateurs, l'absence de changement de phase, l'absence de solvant organique et de déchets solides secondaires à retraiter.

Dans une deuxième modalité de l'invention, la nanofiltration peut être remplacée par, ou combinée à, un autre moyen de séparation, une résine ou support échangeur d'ions d'un type particulier, portant des ligands tels que décrits ci-dessus.

Comme dans la première modalité, des isotopes vont se lier de façon préférentielle aux ligands portés par la résine ou le support, tandis que les isotopes qui ne se seront pas complexés vont traverser la résine ou le support et se retrouver dans le perméat. Des étapes d'élution permettent ensuite d'éluer les isotopes qui se sont complexés.

Par définition, avant une étape d'élution, le rétentat est constitué par les isotopes liés aux ligands et donc immobilisés sur la résine ou le support. Le perméat comprend les isotopes qui n'ont pas été complexés aux ligands.

Après une élution, le perméat correspond par définition à l'éluat.

Pour la réalisation de ces résines ou supports, on préfère utiliser des résines polymères, notamment de type styrène, polyester, polyamide, poly(alcène), polyéther, polyimide ou polyuréthane, ou encore des supports inorganiques greffables tels que silice, alumine et aluminosilicates. On préfère les résines à base de polystyrène et plus particulièrement les copolymères à base de styrène et de divinylbenzène. A titre d'exemple, on peut se référer à l'exemple 8 ci-après ou à R. Garcia et al., 1998, Tethraedron Letters 39 : 8651-8654 décrivant une résine à base d'un dérivé styrénique du DTPA (bis(diéthanolamine)amide, ou de diéthylène triamine pentaacétique acide).

Pour préparer les résines selon l'invention, on peut notamment procéder à la polymérisation du monomère complexant en présence d'un ou plusieurs agents de polymérisation et/ou de réticulation (c'est-à-dire les monomères formateurs de la résine), de préférence de styrène et de divinylbenzène, ce qui conduit à la formation de liaisons covalentes entre le support et la partie complexante du ligand. Ce type de polymère peut aussi être obtenu par greffage sur ce type de résine par formation de liaisons amide, ester, ou éther entre le ligand et la matrice polymérique. Avec un support inorganique la synthèse de résine est de préférence basée sur une méthode de greffage.

Comme dans le cas de la nanofiltration, il est possible d'utiliser plusieurs étages, soit plusieurs colonnes de chromatographie contenant une résine ou un support conforme à l'invention, ces colonnes pouvant être disposées en série et/ou en parallèle, de façon à retraiter le rétentat après élution et/ou le perméat en vue de la séparation des isotopes recherchés et leur enrichissement en fonction du cahier des charges. De même, au cours d'un même procédé, on peut employer différents couples résine (ou support)-ligand de façon à optimiser la séparation des isotopes, notamment lorsque l'on recherche plusieurs isotopes parmi ceux du mélange initial.

On peut avantageusement combiner nanofiltration et passage sur résine ou support. Dans ce cas, on préfère que la ou les étapes de nanofiltration précèdent la ou les étapes sur résine ou support.

L'élution des isotopes complexés peut être réalisée par différentes solutions aqueuses, notamment solution acide, solution de cations ou solution contenant un ligand capable de décrocher les isotopes immobilisés sur la résine tel l'EDTA, le DTPA ou leurs dérivés, notamment ceux définis par la formule (I).

La présente invention décrit un nouveau composé, le N-acétamidostyrène éthylène diamine triacétique acide dont la formule et un procédé de préparation sont indiqués à exemple 7.

L'invention prévoit l'utilisation de ce composé comme ligand dans le procédé de séparation isotopique conforme à l'invention. Elle prévoit également l'utilisation de ce composé en tant que ligand fixé sur une résine ou un support inorganique conforme à invention, pour la réalisation d'une résine ou support échangeur d'ions selon l'invention. Tout particulièrement, l'invention fournit un polymère styrène-divinylbenzène-N-acétamidostyrène éthylène diamine triacétique acide tel que par exemple décrit et produit selon l'exemple 8. Cette résine peut être utilisée pour la séparation isotopique conforme à l'invention.

L'invention va être maintenant décrite plus en détails et à l'aide de modes de réalisation pris à titre d'exemples non limitatifs.

### EXEMPLE 1 : Synthèse du DTPA bis(diéthanolamine)amide (1)

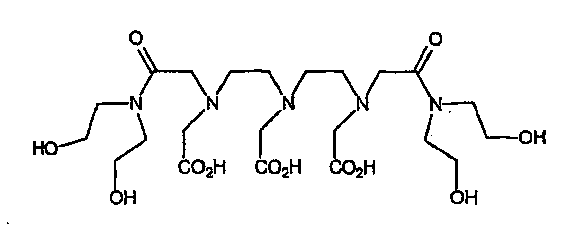

Dans un tricol de 500 mL, 10 grammes d'anhydride de DTPA (27,98 mmol) sont dissous dans 150 mL de DMF (DiMéthylFormamide) anhydre à 80°C sous atmosphère inerte (argon). 17 grammes de diéthanolamine (167,9 mmol) dans 50 mL de DMF sont ajoutés goutte-à-goutte et le milieu réactionnel est maintenu sous agitation pendant 48 heures. Le résidu huileux obtenu est séparé du solvant par décantation. Après dissolution de ce résidu dans le minimum d'eau, 800 mL d'acétone sont additionnés et le précipité visqueux est trituré, isolé du solvant par décantation, et purifié sur colonne de résine échangeuse d'ions Amberlite IR-120 (Fluka) par élution avec de l'eau distillée. On obtient après évaporation et séchage sous vide 7,98g de produit (1) sous forme de poudre blanche (50% de rendement). RMN ¹H (D₂O) : 3,1 (t, J=6,25, 4H): 3,48-3,52 (t+s, 10H): 3,59 (t, J=6,2, 4H); 3,76 (t, J=5,2, 8H); 3,91 (s, 4H), 4,49 (s, 4H).
RMN ¹³C (D₂O) : 50,59, 55,88, 56,62, 58,84, 59,95 (CH₂CO₂H et NCH₂CH₂N); 51,75, 52,14, 60,85, 61,2 (N(CH₂CH₂OH)₂; 169,02, 172,6,176,8 (CO₂H et CO).
ES-MS.: ES⁻ : 566,3 ([M-H]⁻); 282,7 ([M-2H]²⁻/2).

### EXEMPLE 2 : Séparation isotopique du Gd par nanofiltration-complexation avec du DTPA bis(diéthanolamine)amide (1)

L'installation comporte un réacteur de complexation, une pompe, puis un module de filtration plan équipé de la membrane SEPA MG-17 (d'une surface filtrante S=0,015 m²). La membrane plane SEPA MG-17 présente une perméabilité à l'eau bidistillée de 2,5 L.h⁻¹.m⁻².bar⁻¹ à 25°C. La sortie de rétentat est reliée au réacteur de complexation. Cette installation sera utilisée pour tous les exemples de nanofiltration.
Dans cet exemple, on traite une solution aqueuse de volume égal à 1 litre contenant 10 mmol/L de gadolinium (Gd) sous forme de nitrate de gadolinium hexahydraté. On ajoute à la solution aqueuse à traiter un agent complexant constitué par le complexant (1) de l'exemple 1.
On réalise la séparation isotopique du Gd dans les conditions suivantes:
- pression transmembranaire ΔP=0,6 MPa,
- température=20°C,
- débit de rétentat=80 L/h,
- pH=3,8.
On ajoute du complexant (1) à hauteur de 9 mmol/L (soit 90% de ligand par-rapport au gadolinium). On filtre 700 mL de la solution. En fin d'expérience, on obtient un volume de rétentat de 300 mL et un volume de perméat de 700 mL. On prélève un échantillon de perméat et un échantillon de rétentat. Le rétentat final contient 1260 mg/L de Gd, le perméat final en contient 136 mg/L (soit un taux de rétention moyen de 89% au cours de la filtration). L'analyse des deux échantillons par ICP-MS (Inductively Coupled Plasma-Mass Spectroscopy ou spectroscopie de masse couplée à une torche à plasma) fait apparaître les résultats suivants:
- valeur moyenne du rapport ¹⁶⁰Gd/¹⁵⁵Gd dans le rétentat: 1,5086±0,0016
- valeur moyenne du rapport ¹⁶⁰Gd/¹⁵⁵Gd dans le perméat: 1,5128±0,0011
Soit, entre le perméat et le rétentat, un facteur d'enrichissement de 1,0028±0,0018 entre les isotopes ¹⁶⁰Gd et ¹⁵⁵Gd. On a enrichi le perméat en ¹⁶⁰Gd.

### EXEMPLE 3 : Séparation isotopique du Nd par nanofiltration-complexation avec du DTPA bis(diéthanolamine)amide (1)

Dans cet exemple, on traite une solution aqueuse de volume égal à 500 mL contenant 5 mmol/L de néodyme (Nd) sous forme de nitrate de néodyme hexahydraté. On utilise une installation identique à celle de l'exemple 1. On ajoute à la solution aqueuse à traiter un agent complexant constitué par le complexant (1) de l'exemple 1.
On réalise la séparation isotopique du Nd dans les conditions suivantes:
- pression transmembranaire ΔP=0,6 MPa,
- température=20°C,
- débit de rétentat=80 L/h,
- pH=3,8.
On ajoute du complexant (1) à hauteur de 4,5 mmol/L (soit 90% de ligand par rapport au néodyme). On filtre la solution tout en recyclant à la fois les circuits de rétentat et de perméat. À l'équilibre chimique, on prélève un échantillon de perméat et un échantillon de rétentat. Le rétentat contient 613 mg/L de Nd, le perméat en contient 84 mg/L (soit un taux de rétention instantané de 87%). L'analyse des deux échantillons par ICP-MS fait apparaître les résultats suivants:
- valeur moyenne du rapport ¹⁵⁰Nd/¹⁴²Nd dans le perméat: 0,22233±0,00034
- valeur moyenne du rapport ¹⁵⁰Nd/¹⁴²Nd dans le rétentat: 0,22187±0,00040
Soit, entre le perméat et le rétentat, un facteur d'enrichissement de 1,0021±0,0006 entre les isotopes ¹⁵⁰Nd et ¹⁴²Nd. On a enrichi le perméat en ¹⁵⁰Nd.

### EXEMPLE 4 : Synthèse du copolymère DTPA-4,4'-méthylènedianiline (2)

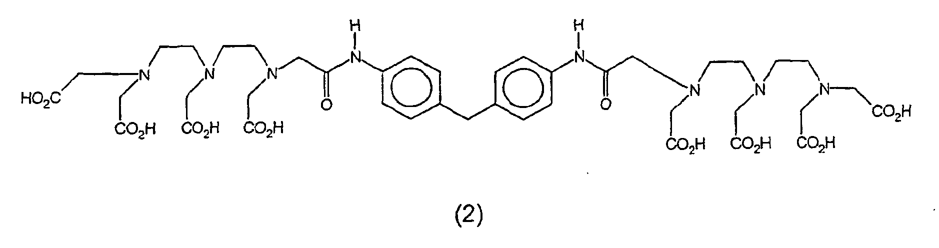

Dans un tricol de 250 mL, 1,179 grammes de dianhydride de DTPA (3,3 mmol) sont dissous dans 120 mL de DMF anhydre à 50°C sous atmosphère inerte (argon). 595 milligrammes de 4,4'-méthylènedianiline (3 mmol) dans 45 mL de DMF anhydre sont ajoutés goutte-à-goutte et le milieu réactionnel est maintenu sous agitation pendant 4 heures à 50°C. Le mélange réactionnel est coulé sur 500 mL d'éther diéthylique. Le précipité est filtré et lavé par 3 fois 100 mL d'éther diéthylique. Après séchage sous vide, on obtient 1,28 g de produit (2) sous forme de poudre blanche (72 % de rendement).
RMN ¹H (D₂O) : 7,17 (s large, 2H) ; 6,76 (m, 2H) ; 3,3-2,1 (m, 14H).
Calcul du degré de polymérisation par RMN ¹H : soit :
R=surface CH aromatiques/surface CH₂ aliphatiques=0,296
n=-18R/(20R-8)=2,57

### EXEMPLE 5 : Synthèse du DTPA bis-(di(2-méthoxyéthyl)amide) (3)

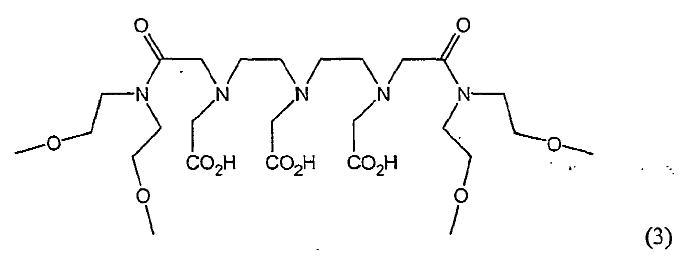

À 5 grammes d'anhydride de DTPA (0,014 mole), dissous dans 80 mL de DMF anhydre sous argon à 80°C, sont ajoutés goutte-à-goutte 12,4 mL de bis(2-méthoxyéthyl)amine (0,084 mole) dissous dans 40 mL de DMF anhydre. Le milieu réactionnel est maintenu sous agitation pendant 24 heures. Après concentration et addition de diéthyléther, le précipité huileux est séparé des solvants par décantation. Ce résidu est dissous dans un minimum de CHCl₃ et reprécipité dans ET₂O. Après séchage sous vide, on obtient une mousse hygroscopique (3) (6,43 g, 74 % de rendement) utilisée sans purification supplémentaire.
RMN¹H (D₂O+NaOD) : 2,50 (t, 4H) ; 2,52 (t, 4H) ; 2,98 (s, 2H) ; 3,11 (s, 4H) ; 3,31 (s, 6H) ; 3,32 (s, 6H) ; 3,51 (s, 4H) ; 3,53-3,56 (m, 16H).
RMN ¹³C (D₂O) : 45,94, 47,03, 47,81, 49,75, 53,69, 56,29, 57,78, 67,16, 69,56, 69,69 (CH₂) ; 58,63, 59,07, (OCH₃) 166,9, 170,5, 175,7 (CO₂H et CO).
ES-MS : ES⁻ : 622,1 ([M-H]⁻) ; ES⁺ : 624,3 ([M+H]⁺) ; 646,2 ([M+Na]⁺).

### EXEMPLE 6 : Synthèse du DTPA bis(1-deoxy-1-amidosorbitol) :

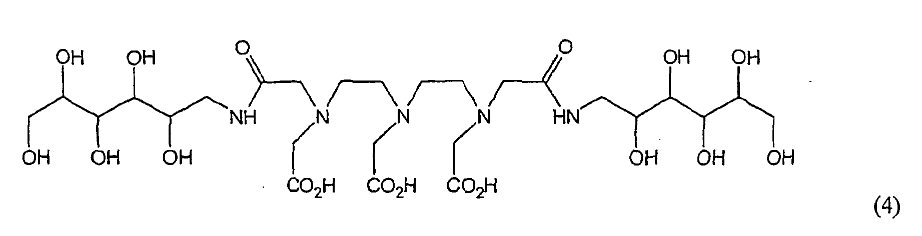

Dans un tricol de 500 mL, 5 grammes d'anhydride de DTPA (0,014 mole) sont dissous dans 100 mL de DMF anhydre à 70°C sous argon. 5,32 grammes de 1-deoxy-1-amino-sorbitol (0,029 mol) dissous dans 40 mL de DMSO sont ajoutés goutte-à-goutte et le milieu réactionnel est maintenu sous agitation pendant 24 heures. Le résidu visqueux issu de la réaction est séparé des solvants après décantation. Il est ensuite dissous dans un minimum d'eau et reprécipité par addition d'acétone. L'opération est répétée une deuxième fois et l'huile résiduelle est séparée par décantation et séchée sous vide pour donner une mousse blanche légèrement teintée (4) (6,66g, 66 % de rendement).
RMN ¹³C (D₂O) : 42,17, 47,14, 49,61, 53,41, 56,73, 57,48, 63,16 (CH₂) ; 69,44, 71,08, 71,18, 71,38 (CH) ; 171,0, 178,86 (CO₂H et CO).
ES-MS : ES⁺ : 741,2 ([M+Na]⁺).

### EXEMPLE 7 : Synthèse du N-acétamidostyrène éthylène diamine triacétique acide

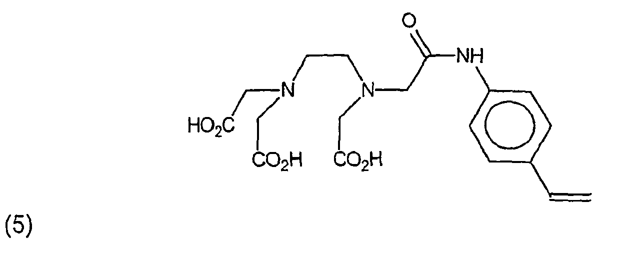

Dans un tricol de 250 mL, 5 grammes de dianhydride d'EDTA (19,5 mmol) sont dissous dans 120 mL de DMF anhydre à 70°C sous atmosphère inerte (argon). 2,09 grammes de 4-vinylaniline (17,57 mmol) dans 20 mL de DMF anhydre sont ajoutés goutte-à-goutte et le milieu réactionnel est maintenu sous agitation pendant 24 heures. La solution est concentrée et le résidu huileux obtenu est trituré dans l'éther éthylique. Un solide rose est obtenu après filtration. Ce solide est mis en suspension dans de l'acétone, puis filtré. Le solide est repris dans de l'eau déionisée, trituré puis filtré. Après séchage sous vide, 4,2 grammes de produit (5) sont obtenus sous forme de poudre rose pâle.
RMN ¹H (DMSO d⁶) (ppm) : 2,8 (s, 4H) ; 3,48 (m, 8H) ; 5,17 (d, 1 H) ; 5,73 (d, 1 H) ; 6,67 (dd, 1 H) ; 7,42 (d, 2H) ; 7,62 (d, 2H).
RMN ¹³C (DMSO d⁶) (ppm) : 51,3 ; 51,4 ; 52,0 ; 54,55 ; 55,1 ; 55,3 ; 58,0 ; 112,7 ; 119 ; 126,5 ; 132,2 ; 136,1 ; 138,5 ; 169,7 ; 172,2 ; 172,9.

### EXEMPLE 8 : Synthèse du polymère styrène-divinylbenzène-N-acétamidostyrène éthylène diamine triacétique acide (6)

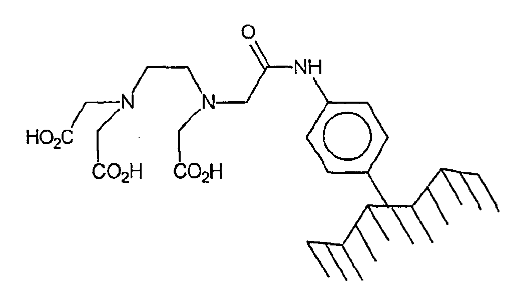

Dans un réacteur en verre, 6 grammes de N-acétamidostyrène éthylène diamine triacétique acide (15,2 mmol) sont mis en solution dans 40 mL de MeOH sous atmosphère inerte. 3,89 grammes de nitrate de sodium (45,7 mmol) broyés sont ajoutés. Cette solution est placée dans le bain à eau d'une cuve à ultrasons et soumise à sonication pendant 15 minutes. 16,5 mL de styrène, 1,49 mL de divinylbenzène et 50 mg d'AIBN (azoisobutyronitrile) sont ajoutés. La solution est agitée et chauffée pendant 72 heures à 65°C. Le solide obtenu est lavé avec MeOH, puis mis en suspension dans un flacon contenant 250 mL de MeOH et agité pendant 24 heures. Le solide est filtré, puis lavé 5 fois avec une solution aqueuse d'acide chlorhydrique (1N), puis 5 fois avec une solution aqueuse de soude (0,1N) et enfin avec de l'eau déionisée jusqu'à pH=5-6. Cette résine est séchée dans un dessicateur contenant du P₂O₅. 15,96 grammes de polymère sont obtenus (72% de rendement).
Analyse élémentaire : trouvée % C : 71,61 ; % H : 6,84 ; % N : 3,65.

### EXEMPLE 9 : Séparation des isotopes du gadolinium en extraction solide-liquide

Dans une colonne chromatographique pour chromatographie liquide haute pression (HPLC) en acier inoxydable dont l'extrémité inférieure est reliée à une pompe, on introduit une suspension de 5,5 grammes de polymère styrène-divinylbenzène-(N-acétamidostyrène) (6) tamisé (taille des particules de 108 à 300 µm) dans une solution aqueuse de nitrate de sodium (C=1×10⁻² mol.L⁻¹). La colonne est soumise à des vibrations afin de tasser le polymère et éviter la formation de bulles d'air. Une fois remplie, la colonne est fermée hermétiquement, puis montée en série avec une pompe utilisée en chromatographie liquide haute pression. Une solution de nitratre de sodium (C=1×10⁻² mol.L⁻¹) est éluée afin de vérifier que la pression reste constante et afin de régler le débit (débit=0,2 mL.min⁻¹, pression=12 bars). Enfin, 1 L d'une solution aqueuse de nitrate de gadolinium est élué (C=1×10⁻² mol/L). La solution en sortie de colonne est alors fractionnée en échantillons de 11 mL. Ces différentes fractions sont dosées en ICP-AES afin de déterminer la teneur de chaque échantillon en sodium et en gadolinium. L'analyse de trois échantillons (solution initiale et deux solutions correspondant à des volumes ajoutés de 220 mL (S₂₀) et 275 mL (S₂₅) de Gd(NO₃)₃.6H₂O) par ICP-MS fait apparaître les résultats suivants:
- valeur moyenne du rapport ¹⁶⁰Gd/¹⁵⁵Gd dans la solution initiale: 1,52505±0,00107
- valeur moyenne du rapport ¹⁶⁰Gd/¹⁵⁵Gd dans la solution S₂₀: 1,51297±0,00417
- valeur moyenne du rapport ¹⁶⁰Gd/¹⁵⁵Gd dans la solution S₂₅: 1,51883±0,00222
Soit des facteurs d'enrichissement pour Sᵢₙᵢₜᵢₐₗₑ/S₂₀=1,0080±0,0035 entre les isotopes ¹⁶⁰Gd et ¹⁵⁵Gd et Sᵢₙᵢₜᵢₐₗₑ/S₂₅=1,0041±0,0022 entre les isotopes ¹⁶⁰Gd et ¹⁵⁵Gd.
Les isotopes légers sortent en premier de la colonne (rétention sélective des isotopes lourds dans la colonne).

## Revendications

1. Procédé pour séparer, en milieu aqueux, des isotopes d'un même élément, appartenant aux lanthanides, procédé comprenant le traitement d'un milieu aqueux comprenant des isotopes d'un élément, sous forme de sel avec un ligand organique ayant dans le milieu aqueux le même nombre de charge négatives que l'isotope sous forme ionique a de charges positives, ce ligand étant conçu pour se lier préférentiellement à un ou certains isotopes de l'élément, procédé dans lequel soit (i) le ligand est porté par une résine ou support échangeur d'ions, le milieu aqueux étant mis en contact avec la résine ou support, les isotopes se liant aux ligands formant un rétentat tandis que les isotopes non retenus traversent la résine ou support soit (ii) le ligand, hydrosoluble, est mis en solution dans le milieu aqueux et le complexe ligand-isotope formé est retenu par une membrane de nanofiltration, et l'on sépare dans chaque cas (i) et (ii) un rétentat comprenant les isotopes qui se sont complexés au ligand, et un perméat comprenant les isotopes qui ne se sont pas complexés.

2. Procédé selon la revendication 1, **caractérisé en ce que** le ligand comprend un polyacide aminé linéaire ou cyclique, de préférence linéaire, répondant à la formule (1) suivante : 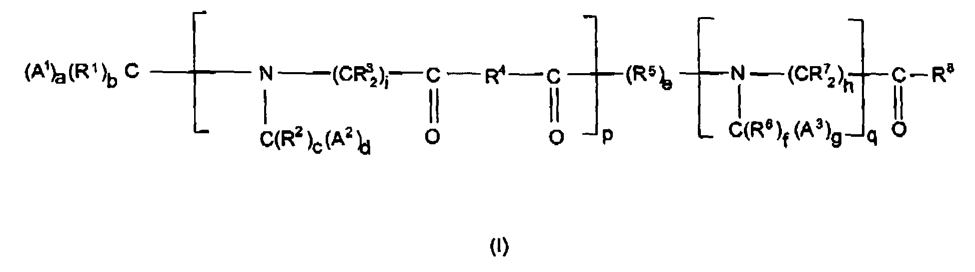 dans laquelle :
- a = 0 ou 1 et b = 2 ou 3,
- c = 2 ou 3 et d = 0 ou 1,
- p = 0 à 3.
- e = 0 ou 1,
- h = 1,2 ou 3,
- i = 1,2 ou 3,
- q = 1 à 4,
- f = 2 ou 3,
- g = 0 ou 1,
A¹, A², A³ sont identiques ou différents entre eux et correspondent à un groupement acide monovalents;
- les R₁ sont identiques ou différents entre eux et correspondent à :
∈ H,
alkyle en C₁-C₁₀ ou,
∈ 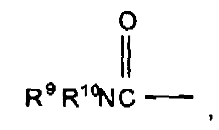 avec a = 0, b = 1 et R⁹, R¹⁰ identiques ou différents et correspondant chacun à l'hydrogène ou à un radical monovalent hydrophile ;
- les R² sont identiques ou différents entre eux,
les R³ sont identiques ou différents entre eux,
les R⁶ sont identiques ou différents entre eux,
les R⁷ sont identiques ou différents entre eux,
R², R³, R⁶, R⁷ sont identiques ou différents entre eux et correspondent à H ou à un alkyle en C₁-C₁₀;
- les R⁴ sont identiques ou différents entre eux et correspondent à un groupement divalent hydrophile;
- le groupement divalent R⁵ représentant un alkylène ou un groupement répondant à la même définition que R⁴ ; et/ou
- le groupement R⁸ correspondant à un hydroxyle, à A⁴ répondant à la même définition que A¹, A², A³, à l'hydrogène ou à -NR⁹R¹⁰ avec R⁹, R¹⁰ identiques ou différents entre eux et représentant un radical monovalent hydrophile.

3. Procédé selon la revendication 2, dans lequel p est 2.

4. Procédé selon la revendication 2, dans lequel h est 1 ou 2.

5. Procédé selon la revendication 2, dans lequel i est 1 ou 2.

6. Procédé selon la revendication 2, dans lequel q est 2 ou 3.

7. Procédé selon la revendication 2, dans lequel A¹, A² et A³ sont sélectionnés dans le groupe comportant :-COOR, -PO₃R'₂, -SO₃R", avec R, R', R" = H ou cation.

8. Procédé selon la revendication 7, dans lequel R, R', R" = H ou cation alcalin.

9. Procédé selon la revendication 2, dans lequel R⁹ et R¹⁰ sont sélectionnés parmi les restes hydrocarbonés aminés et/ou (poly)hydroxylés et/ou alcoxylés et/ou (poly)étherifiés.

10. Procédé selon la revendication 9, dans lequel ces restes sont du type (cyclo)alkyle, aralkyle, alkylaryle, (cyclo)alcényle, aralcényle, alcénylaryle, aryle.

11. Procédé selon la revendication 2, dans lequel R⁹, R¹⁰ correspondent chacun à un hydroxyalkyle en C₁-C₁₀, un alcoxyle en C₁-C₁₀, un polyol.

12. Procédé selon la revendication 2, dans lequel R⁴ est choisi parmi les groupements aminés et/ou hydroxylés aromatiques, les groupements aminés et/ou hydroxylés aromatiques et alkyles, les groupements aminés et/ou hydroxylés aromatiques et (cyclo)alkylèniques, ou les groupements aminés et/ou hydroxylés (cyclo)alkyléniques.

13. Procédé selon la revendication 2 ou 12, dans lequel R⁴ comporte des alcoxyles et/ou des (poly)éthers.

14. Procédé selon la revendication 2, dans lequel R⁵ est CH₂.

15. Procédé selon la revendication 2, dans lequel R⁸ est un hydroxyalkyle en C₁-C₁₀, un alcoxyle en C₁-C₁₀ ou un polyol.

16. Procédé selon la revendication 15, dans lequel R⁸ est un saccharide hydrogéné.

17. Procédé selon la revendication 2, dans lequel les groupements hydrophile R⁹ et/ou R¹⁰ sont des polyhydroxyalkyles.

18. Procédé selon la revendication 17, dans lequel R⁹ et/ou R¹⁰ sont des saccharides hydrogénés.

19. Procédé selon la revendication 18, dans lequel R⁹ et/ou R¹⁰ sont des restes sorbitol.

20. Procédé selon la revendication 17, dans lequel R⁹ et/ou R¹⁰ sont des chaînes polyéthers.

21. Procédé selon la revendication 20, dans lequel R⁹ et/ou R¹⁰ sont des chaînes polyéthylèneglycol ou polypropylèneglycol.

22. Procédé selon l'une quelconque des revendications 2 à 21, dans lequel les groupements alkyles, alcoxyles et/ou alcényles sont en C₂, C₃ et/ou C₄.

23. Procédé selon la revendication 2, dans lequel le ligand est un composé de formule (I.1) : 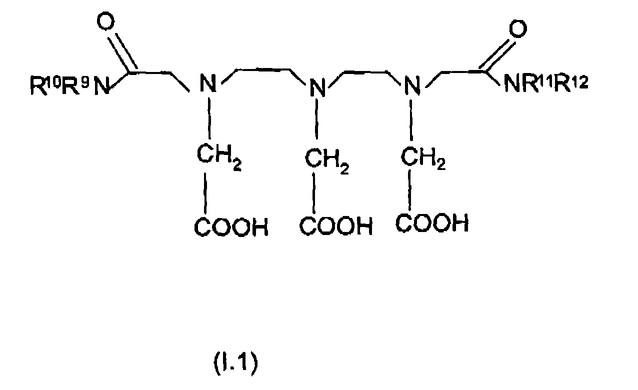 dans laquelle R³, R¹⁰, R¹¹, R¹² sont identiques ou différents entre eux et représentent chacun un radical monovalent hydrophile répondant à la même définition que celle donnée pour R⁹, R¹⁰ à l'une quelconque des revendications précédentes.

24. Procédé selon la revendication 23, dans lequel R⁹, R¹⁰, R¹¹ et/ou R¹² sont choisis parmi les radicaux éthanoyle, méthoxyéthyle et sorbitoyle.

25. Procédé selon la revendication 1, dans lequel le ligand est le N-acétamidostyrène éthylènediamine triacétique acide de formule :

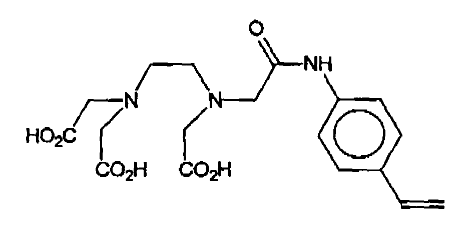

26. Procédé selon la revendication 25, dans lequel le ligand est lié à une résine polymère ou support inorganique.

27. Procédé selon la revendication 26, utilisant comme résine un polymère styrène-divinylbenzène-N-acétamidostyrène éthylène diamine triacétique acide.

28. Procédé selon l'une quelconque des revendications 1 à 27, dans lequel la quantité de ligand employée est inférieure à 1 équivalent de ligand par atome d'isotope ou d'isotopes à complexer.

29. Procédé selon l'une quelconque des revendications 1 à 28, dans lequel le milieu aqueux traité par le ligand est soumis à une nanofiltration.

30. Procédé selon la revendication 29, dans lequel la nanofiltration est une nanofiltration tangentielle.

31. Procédé selon l'une quelconque des revendications 1 à 28, dans lequel l'on fait passer le milieu aqueux comportant les isotopes à travers une résine ou support échangeur d'ions portant le ligand.

32. Procédé selon les revendications 30 ou 31, dans lequel le milieu aqueux est traité par filtration et par résine ou support échangeur d'ions portant le ligand.

33. Procédé selon l'une quelconque des revendications 1 à 32, appliqué à la séparation d'isotopes d'un élément choisi parmi gadolinium, erbium et néodyme.

34. Procédé selon l'une quelconque des revendications 1 à 33, dans lequel le sel de lanthanide est un nitrate, un sulfate, un carbonate ou un chlorure de lanthanide.

## Claims

1. Process for separating, in an aqueous medium, isotopes of the same element belonging to the lanthanide series, said process comprising treatment of an aqueous medium containing isotopes of an element in salt form with an organic ligand, which in the aqueous medium has the same number of negative charges as the isotope in ionic form has positive charges, wherein this ligand is designed to preferentially bind to one or certain isotopes of the element, and in said process either (i) the ligand is carried by a resin or ion-exchange support, wherein the aqueous medium is brought into contact with the resin or support and the isotopes binding to the ligands form a retentate whereas the isotopes not retained pass through the resin or support; or (ii) the ligand, which is hydrosoluble, is put into solution in the aqueous medium and the ligand-isotope complex formed is retained by a nanofiltration membrane, and in each of cases (i) and (ii) a retentate containing the isotopes that are complexed to the ligand is separated and a permeate containing the ligands that are not complexed is separated.

2. Process according to claim 1, **characterised in that** the ligand contains a linear or cyclic, preferably linear, aminated polyacid having the following formula (I): 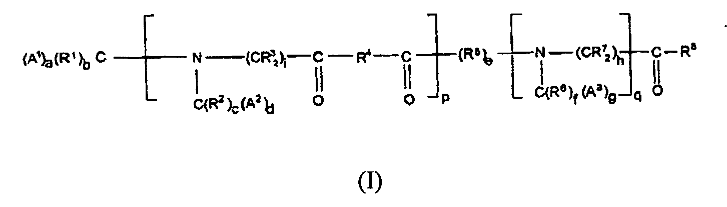 wherein:
• a = 0 or 1 and b = 2 or 3,
• c = 2 or 3 and d = 0 or 1,
• p = 0 to 3,
• e = 0 or 1,
• h = 1,2 or 3,
• i = 1,2 or 3,
• q = 1 to 4,
• f = 2 or 3,
• g = 0 or 1,
A¹, A², A³ are identical or different from one another and correspond to a monovalent acid group;
• R₁ are identical or different from one another and correspond to:
∈ H
alkyl in C₁-C₁₀ or,
∈ 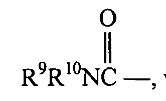
where a = 0, b = 1 and R⁹, R¹⁰ are identical or different and each correspond to hydrogen or a hydrophilic monovalent radical;
• R² are identical or different from one another,
R³ are identical or different from one another,
R⁶ are identical or different from one another,
R⁷ are identical or different from one another,
R², R³, R⁶, R⁷ are identical or different from one another and correspond to H or an alkyl in C₁-C₁₀;
• R⁴ are identical or different from one another and correspond to a hydrophilic divalent group;
• the divalent group R⁵ represents an alkylene or a group having the same definition as R⁴; and/or
• the R⁸ group corresponds to a hydroxyl, to A⁴ having the same definition as A¹, A², A³, to hydrogen or to -NR⁹R¹⁰, wherein R⁹, R¹⁰ are identical or different from one another and represent a hydrophilic monovalent radical.

3. Process according to claim 2, wherein p is 2.

4. Process according to claim 2, wherein h is 1 or 2.

5. Process according to claim 2, wherein i is 1 or 2.

6. Process according to claim 2, wherein q is 2 or 3.

7. Process according to claim 2, wherein A¹, A² and A³ are selected from the group comprising: -COOR, -PO₃R'₂, -SO₃R", where R, R' R" = H or cation.

8. Process according to claim 7, wherein R, R', R" = H or alkaline cation.

9. Process according to claim 2, wherein R⁹ and R¹⁰ are selected from among aminated and/or (poly)hydroxylated and/or alkoxylated and/or (poly)etherified hydrocarbon residues.

10. Process according to claim 9, wherein these residues are (cyclo)alkyl, aralkyl, alkylaryl, (cyclo)alkenyl, aralkenyl, alkenylaryl, aryl.

11. Process according to claim 2, wherein R⁹, R¹⁰ each correspond to a hydroxyalkyl in C₁-C₁₀, an alkoxyl in C₁-C₁₀, a polyol.

12. Process according to claim 2, wherein R⁴ is selected from among aminated and/or aromatic hydroxylated groups, aminated and/or aromatic hydroxylated and alkyl groups, aminated and/or aromatic hydroxylated and (cyclo)alkylene groups, or aminated and/or aromatic hydroxylated (cyclo)alkylene groups.

13. Process according to claim 2 or 12, wherein R⁴ comprises alkoxyls and/or (poly)ethers.

14. Process according to claim 2, wherein R⁵ is CH₂.

15. Process according to claim 2, wherein R⁸ is a hydroxyalkyl in C₁-C₁₀, an alkoxyl in C₁-C₁₀ or a polyol.

16. Process according to claim 15, wherein R⁸ is a hydrogenated saccharide.

17. Process according to claim 2, wherein the hydrophilic groups R⁹ and/or R¹⁰ are polyhydroxyalkyls.

18. Process according to claim 17, wherein R⁹ and/or R¹⁰ are hydrogenated saccharides.

19. Process according to claim 18, wherein R⁹ and/or R¹⁰ are sorbitol residues.

20. Process according to claim 17, wherein R⁹ and/or R¹⁰ are polyether chains.

21. Process according to claim 20, wherein R⁹ and/or R¹⁰ are polyethylene glycol or polypropylene glycol chains.

22. Process according to any one of claims 2 to 21, wherein the alkyl, alkoxyl and/or alkenyl groups are in C₂, C₃ and/or C₄.

23. Process according to claim 2, wherein the ligand is a compound of formula (I.1): 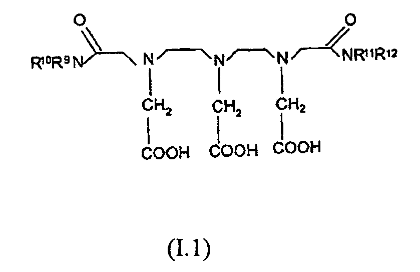 wherein R⁹, R¹⁰, R¹¹, R¹² are identical or different from one another and each represents a hydrophilic monovalent radical having the same definition as that given for R⁹, R¹⁰ in any one of the preceding claims.

24. Process according to claim 23, wherein R⁹, R¹⁰, R¹¹ and/or R¹² are selected from among ethanoyl, methoxyethyl and sorbitoyl radicals.

25. Process according to claim 1, wherein the ligand is N-acetamido styrene ethylene diamine triacetic acid of the formula:

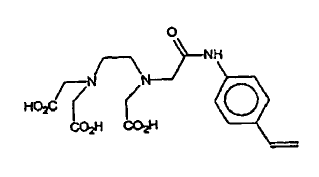

26. Process according to claim 25, wherein the ligand is bound to a polymer resin or inorganic support.

27. Process according to claim 26, using a styrene-divinylbenzene-N-acetamido styrene ethylene diamine triacetic acid polymer as resin.

28. Process according to any one of claims 1 to 27, wherein the quantity of ligand used is lower than 1 ligand equivalent per atom of isotope or isotopes to be complexed.

29. Process according to any one of claims 1 to 28, wherein the aqueous medium treated by the ligand is subjected to a nanofiltration.

30. Process according to claim 29, wherein the nanofiltration is a tangential nanofiltration.

31. Process according to any one of claims 1 to 28, wherein the aqueous medium comprising the isotopes is passed through a resin or ion-exchange support carrying the ligand.

32. Process according to claims 30 or 31, wherein the aqueous medium is treated by filtration and by resin or ion-exchange support carrying the ligand.

33. Process according to any one of claims 1 to 32, applied to the separation of isotopes of an element selected from among gadolinium, erbium and neodymium.

34. Process according to any one of claims 1 to 33, wherein the lanthanide salt is a lanthanide nitrate, sulphate, carbonate or chloride.

## Patentansprüche

1. Verfahren zum Trennen, im wässrigen Milieu, von Isotopen eines gleichen Elements, das zu den Lanthanoiden gehört, wobei das Verfahren die Behandlung eines wässrigen Milieus umfasst, das die Isotopen eines Elements in Form des Salzes mit einem organischen Liganden enthält, wobei im wässrigen Milieu die gleiche Anzahl an negativen Ladungen vorhanden ist, wie das Isotop in ionischer Form als positive Ladungen aufweist, wobei dieser Ligand so entworfen ist, um sich vorzugsweise an eines oder bestimmte Isotope des Elements zu binden, wobei in dem Verfahren entweder (i) der Ligand durch ein Harz oder einen lonenaustauscherträger getragen wird und das wässrige Milieu mit dem Harz oder Träger in Kontakt gebracht wird, die Isotope sich an die Liganden binden und ein Retentat bilden, während die nicht zurückgehaltenen Isotope das Harz oder den Träger durchqueren, oder (ii) der wasserlösliche Ligand in dem wässrigen Milieu gelöst und der gebildete Ligand-Isotop-Komplex durch eine Nanofiltrationsmembran zurückgehalten wird, und in jedem Fall (i) und (ii) das die Isotopen, die mit dem Liganden einen Komplex gebildet haben, enthaltende Retentat von einem die nicht komplexierten Isotopen enthaltenden Permeat getrennt wird.

2. Verfahren nach Anspruch 1, **dadurch gekennzeichnet, dass** der Ligand eine lineare oder cyclische, vorzugsweise lineare, aminierte Polysäure umfasst, die der folgenden Formel (I) entspricht: 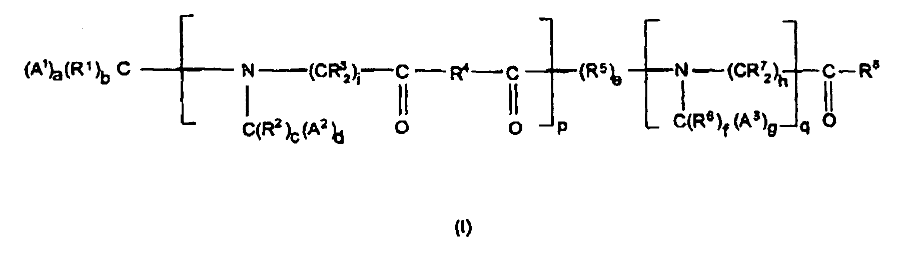 wobei:
- a = 0 oder 1 und b = 2 oder 3,
- c = 2 oder 3 und d = 0 oder 1,
- p = 0 bis 3,
- e = 0 oder 1,
- h = 1, 2 oder 3,
- i = 1, 2 oder 3,
- q = 1 bis 4,
- f = 2 oder 3,
- g = 0 oder 1,
A¹, A², A³ identisch oder voneinander verschieden sind und einer einwertigen Säuregruppe entsprechen;
- die R¹ identisch oder voneinander verschieden sind und folgendem entsprechen;
- H,
- C₁-C₁₀-Alkyl oder
- 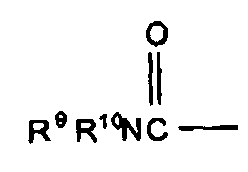 wobei a = 0, b = 1 und R⁹, R¹⁰ identisch oder verschieden sind und jeweils Wasserstoff oder einem einwertigen hydrophilen Rest entsprechen;
- die R² identisch oder voneinander verschieden sind;
die R³ identisch oder voneinander verschieden sind;
die R⁶ identisch oder voneinander verschieden sind;
die R⁷ identisch oder voneinander verschieden sind;
R², R³, R⁶, R⁷ identisch oder voneinander verschieden sind und H oder einem C₁-C₁₀-Alkyl entsprechen;
- die R⁴ identisch oder voneinander verschieden sind und einer zweiwertigen hydrophilen Gruppe entsprechen;
- die zweiwertige Gruppe R⁵ ein Alkylen oder eine Gruppe darstellt, die der gleichen Definition wie R⁴ entspricht; und/oder
- die Gruppe R⁸ einem Hydroxyl, einem A⁴, das der gleichen Definition wie A¹, A², A³ entspricht, Wasserstoff oder -NR⁹R¹⁰ entspricht, wobei R⁹, R¹⁰ identisch oder voneinander verschieden sind und einen einwertigen hydrophilen Rest darstellen.

3. Verfahren nach Anspruch 2, wobei p 2 ist.

4. Verfahren nach Anspruch 2, wobei h 1 oder 2 ist.

5. Verfahren nach Anspruch 2, wobei i 1 oder 2 ist.

6. Verfahren nach Anspruch 2, wobei q 2 oder 3 ist.

7. Verfahren nach Anspruch 2, in welchem A¹, A² und A³ aus der Gruppe bestehend aus -COOR, -PO₃R'₂, -SO₃R" ausgewählt werden, wobei R, R', R" = H oder Kation ist.

8. Verfahren nach Anspruch 7, wobei R, R', R" = H oder ein alkalisches Kation ist.

9. Verfahren nach Anspruch 2, in welchem R⁹ und R¹⁰ aus den aminierten und/oder (poly)hydroxylierten und/oder alkoxylierten und/oder (poly)veretherten Kohlenwasserstoffresten ausgewählt werden.

10. Verfahren nach Anspruch 9, in welchem diese Reste vom Typ (Cyclo)alkyl, Aralkyl, Alkylaryl, (Cyclo)alkenyl, Aralkenyl, Alkenylaryl, Aryl sind.

11. Verfahren nach Anspruch 2, in welchem R⁹, R¹⁰ jeweils einem C₁-C₁₀-Hydroxyalkyl, einem C₁-C₁₀-Alkoxyl oder einem Polyol entsprechen.

12. Verfahren nach Anspruch 2, in welchem R⁴ aus den aminierten und/oder aromatischen hydroxylierten Gruppen, den aminierten und/oder aromatischen hydroxylierten und Alkylgruppen, den aminierten und/oder hydroxylierten aromatischen und (Cyclo)alkylengruppen oder den aminierten und/oder hydroxylierten (Cyclo)alkylengruppen ausgewählt wird.

13. Verfahren nach Anspruch 2 oder 12, in welchem R⁴ Alkoxyle und/oder (Poly)ether umfasst.

14. Verfahren nach Anspruch 2, wobei R⁵ CH₂ ist.

15. Verfahren nach Anspruch 2, in welchem R⁸ jeweils einem C₁-C₁₀-Hydroxyalkyl, einem C₁-C₁₀-Alkoxyl oder einem Polyol entspricht.

16. Verfahren nach Anspruch 15, wobei R⁶ ein hydriertes Saccharid ist.

17. Verfahren nach Anspruch 2, in welchem die hydrophilen Gruppen R⁹ und/oder R¹⁰ Polyhydroxyalkyle sind.

18. Verfahren nach Anspruch 17, wobei R⁹ und/oder R¹⁰ hydrierte Saccharide sind.

19. Verfahren nach Anspruch 18, wobei R⁹ und/oder R¹⁰ Sorbitreste sind.

20. Verfahren nach Anspruch 17, wobei R⁹ und/oder R¹⁰ Polyetherketten sind.

21. Verfahren nach Anspruch 20, wobei R⁹ und/oder R¹⁰ Polyethylenglykol- oder Polypropylenglykolketten sind.

22. Verfahren nach einem der Ansprüche 2 bis 21, in welchem die Alkyl-, Alkoxyl- und/oder Alkenylgruppen C₂-, C₃- und/oder C₄-Gruppen sind.

23. Verfahren nach Anspruch 2, in welchem der Ligand eine Verbindung der Formel (I.1) ist: 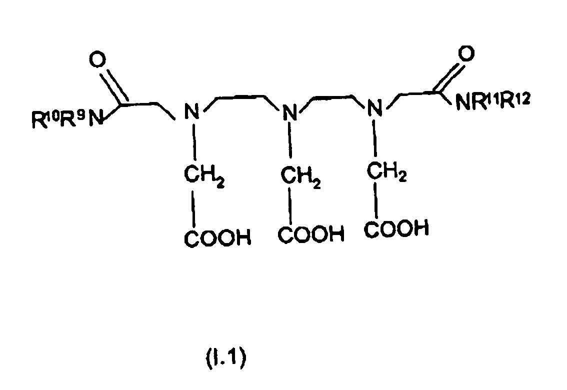 wobei R⁹, R¹⁰, R¹¹, R¹² identisch oder voneinander verschieden sind und jeweils einen einwertigen hydrophilen Rest darstellen, welcher der gleichen Definition entspricht, die für R⁹, R¹⁰ in einem der vorhergehenden Ansprüchen gegeben wurde.

24. Verfahren nach Anspruch 23, in welchem R⁹, R¹⁰, R¹¹ und/oder R¹² aus Ethanoyl-, Methoxyethyl- und Sorbitoylresten ausgewählt werden.

25. Verfahren nach Anspruch 1, in welchem der Ligand N-Acetamidostyrol-ethylendiamin-triessigsäure der folgenden Formel ist:

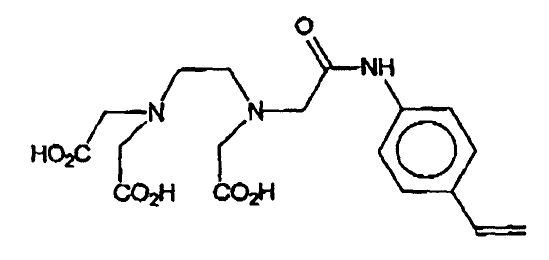

26. Verfahren nach Anspruch 25, in welchem der Ligand an ein Polymerharz oder einen anorganischen Träger gebunden ist.

27. Verfahren nach Anspruch 26, wobei als Harz ein Styrol-Divinylbenzol-N-Acetamidostyrol-ethylendiamin-triessigsäure-Polymer verwendet wird.

28. Verfahren nach einem der Ansprüche 1 bis 27, in welchem die Menge an eingesetztem Liganden weniger als 1 Äquivalent Ligand pro Atom des oder der zu komplexierenden Isotope beträgt.

29. Verfahren nach einem der Ansprüche 1 bis 28, in welchem das mit dem Liganden behandelte, wässrige Milieu einer Nanofiltration unterzogen wird.

30. Verfahren nach Anspruch 29, in welchem die Nanofiltration eine tangentielle Nanofiltration ist.

31. Verfahren nach einem der Ansprüche 1 bis 28, in welchem das die Isotope enthaltende wässrige Milieu durch ein Harz oder einen Ionenaustauscherträger, der den Liganden trägt, geschickt wird.

32. Verfahren nach einem der Ansprüche 30 bis 31, in welchem das wässrige Milieu mittels Filtration und mittels des Harzes oder des lonenaustauscherträgers, der den Liganden trägt, behandelt wird.

33. Verfahren nach einem der Ansprüche 1 bis 32, anwendbar auf die Trennung von Isotopen eines Elements ausgewählt aus Gadolinium, Erbium und Neodym.

34. Verfahren nach einem der Ansprüche 1 bis 33, in welchem das Lanthanoidsalz ein Lanthanoidnitrat, -sulfat, -carbonat oder -halogenid ist.
